# EUROPEAN PATENT APPLICATION

(11) **EP 2 077 274 A1**
(43) Date of publication of application: **08.07.2009**
(21) Application number: 08000005.2
(22) Date of filing: 02.01.2008
(51) Int. Cl.: C07K 14/47, A61K 38/00

(54) **Synthetic analogs of human beta-defensins having antimicrobial, antiviral and chemotactic activity**

(71) Applicant: Ceinge Biotecnologie Avanzate s.c. a r.l., 80145 Napoli (IT)
(72) Inventor: Castaldo, Giuseppe, 80145 Napoli (IT); Galdiero, Stefania, 80145 Napoli (IT); Pedone, Carlo, 80145 Napoli (IT); Salvatore, Francesco, 80145 Napoli (IT); Scudiero, Olga, 80145 Napoli (IT)
(74) Representative: Minoja, Fabrizio

(57) **Abstract**

The invention concerns peptides having sequences corresponding to fragments of human beta defensins 1 and/or 3 having antibacterial and/or antiviral activity, even in the presence of high sodium chloride concentration.

## Description

The invention concerns peptides having sequences corresponding to fragments of human beta defensins 1 and/or 3 having antibacterial and/or antiviral activity, even in the presence of high sodium chloride concentration.

### Background of the invention

Two families of defensins have been described in mammals, i.e., alpha and beta, classified on the basis of sequence homology and disulfide bonds positions. Human beta-defensins (hBDs)⁹ are highly conserved small peptides produced by plants, insects, invertebrates and vertebrates, developed as a part of primordial immune protective mechanism (1). A computational strategy revealed five conserved gene clusters containing about 30 hBD genes in humans (2). A cluster of five hBD genes mainly expressed in epididymis was identified on chromosome 20 (3). The biological activities of four hBD proteins encoded by genes belonging to one of the five gene clusters and located on human chromosomal region 8p21-p23 are known. These peptides, called hBD1 (DEFB1), hBD2 (DEFB4), hBD3 (DEFB103A), hBD4 (DEFB104) are expressed mainly by respiratory, gastrointestinal and urogenital epithelial cells (1), either constitutively (hBD1) or after induction by microorganisms or inflammatory factors (hBD2-4). Human BDs 1, 2 and 4 are highly polymorphic, also due to hBD2 and 4 gene duplications, whereas only rare gene variants have been identified within the hBD3 gene (4).

All these four beta-defensins are cationic, 36-45 amino acids long and show a similar folding and an invariable six-cysteine motif that gives rise to three disulfide bonds (5-9). The four human beta-defensins exert different prochemotactic, antineoplastic and immunomodulating activities, in addition to bactericidal and antiviral activity toward various human pathogens (10-12). The antibacterial activity of hBD1 (13), which is predominantly active toward *Pseudomonas aeruginosa* and other gram negatives, of hBD2 (14) and of hBD4 (15) is inhibited by the enhanced NaCl concentration typically present in the airway surface fluid (ASF) of cystic fibrosis (CF) patients. On the contrary, hBD3 has been reported to be insensitive to NaCl (16), due to its different structural characteristics and charge, even if the study has been performed only up to 150 mM NaCl (17), whereas the salt concentrations at airway surface of CF patients is higher (18).

Cystic fibrosis (CF) is the most frequent (incidence: 1:2500) life-shortening autosomal recessive disease among Caucasians (19). It depends on mutations of the Cystic Fibrosis Transmembrane Regulator (CFTR) gene that encodes a ubiquitous chloride channel. The altered activity of the CFTR protein causes the production of hyperosmolar viscous mucus particularly at pulmonary level (19, 20) and consequently respiratory colonization by *P. aeruginosa* and other multi-resistant strains (19-22). In almost 95% of CF patients the cause of death is respiratory insufficiency due to chronic neutrophilic inflammation (22, 23). Beta-defensins exert also chemotactic activity: hBD1, 2 and 3 toward monocytes, dendritic and T cells, and hBD3 mainly towards macrophages (1, 24, 25). On the contrary, conflicting data have been reported on hBD1 chemotactic activity toward granulocytes (24, 25). Furthermore, the chemotactic activity of hBD1 is not impaired at physiological NaCl concentration (26), while it is not yet known whether the enhanced NaCl concentration in CF patients modulates the chemotactic activity of hBDs.

There is some preliminary evidence that hBDs exert antiviral activity. Human beta-defensin 2 and 3 inhibit human immunodeficiency virus (HIV)-1 replication and virion infectivity (27, 28), and they further modulate HIV-1 co-receptor expression (28). Human herpes simplex virus (HSV)-1 affects over 50% of the European population and is very contagious: no vaccines are yet available, although some nucleosides exert therapeutic effects (29). Nevertheless, the emergence of HSV-resistant strains and the failure to completely relieve symptoms have prompted the quest for antivirals. HSV-1 and 2 and other viruses, preincubated with alpha (hNP)1-3 (30, 31) or theta (32) defensins loose their ability to infect target cells (31). On the contrary, there are no data about hBD activity toward HSV-1 and 2.

### Description of the invention

The invention concerns peptides having sequences corresponding to fragments of human beta defensins 1 and/or 3 having antibacterial and/or antiviral activity, independently on the presence of high sodium chloride concentration. The peptides of the invention also exhibit chemotactic activity and may be used as active ingredients of pharmaceutical compositions for the treatment and prevention of bacterial or viral infections, particularly in patients affected by cystic fibrosis.

The sequences of the peptides correspond to that of fragments of human beta defensin 1 or of human beta defensin 3 and preferably comprise at least a fragment of human beta defensin 1 (hBD 1) fused to a fragment of human beta defensin 3 (hBD3).

The term "fragment" refers to a peptide sequence, usually consisting of 5 to 22 amino acids, aligned to the sequences of human beta defensins 1 or 3.

The amino acid sequence of hBD1 and hBD3 are reported below:
**hBD1** DHYNCVSSGGQCLYSACPIFTKIQGTCYRGKAKCCK (SEQ ID 1)
**hBD3** GIINTLQKYYCRVRGGRCAVLSCLPKEEQIGKCSTRGRKCCRRKK (SEQ ID 2).

The peptides of the invention comprise from 5 to 40 amino acids, preferably from 8 to 42 amino acids, even more preferably from 12 to 39 amino acids.

Preferably, the fragments include sequences of the C terminal of human beta defensin 3 or of human beta defensin 1, and/or sequences from the internal region of human beta defensin 1.

Specific examples of peptides of the invention are reported below:
- DHYNCVSSGGQCLYSACPIFTKIQGTCYRGKRKCCRRKK (SEQ ID 3)
- GIINTLDHYNCVSSGGQCLYSACPIFTKIQGTCYRGKAKCCK (SEQ ID 4)
- GIINTLQKYYCRVRGGRCAVLSCLPKEEQIGKCSTRGAKCCK (SEQ ID 5)
- QKYYCRVRGGRCAVLSCLPKEEQIGKCSTRGRKCCRRKK (SEQ ID 6)
- DHYNCVSSGGQCLYSACLPKEEQIGKCYRGKAKCCK (SEQ ID 7)
- GIINTLQKYYCRVRGGRCAVLSCPIFTKIQGTCSTRGRKCCRRKK (SEQ ID 8)
- DHYNCVSSGGQCLYSACLPKEEQIGKCYRGKRKCCRRKK (SEQ ID 9)
- GIINTLQKYYCRVRGGRCAVLSCPIFTKIQGTCSTRGAKCCK (SEQ ID 10)
- GIINTLQKYYCRVRGGRCAVLSCLPKQQQIGKCSTRGRKCCRRKK (SEQ ID 11)
- DHYNCVSSGG (SEQ ID 12)
- GIINTLQKYYCRVRGGRCAVLS (SEQ ID 13)
- QKYYCRVRGGRCAVLS (SEQ ID 14)
- IFTKIQGTAKCCK (SEQ ID 15)
- AKCCK (SEQ ID 16)
- IFTKIQGTRKCCRRKK (SEQ ID 17)
- RKCCRRKK (SEQ ID 18)
- IFTKIQGTKCCRRKK (SEQ ID 19)
- IFTKIQGT (SEQ ID 20).

Peptides having the sequences SEQ ID 3, SEQ ID 6, SEQ ID 8 and SEQ ID 9 are particularly preferred.

The invention also includes derivatives and analogues of said peptides wherein one or more natural L- amino acids in the sequences have been substituted with D-amino acids or mutated by deletion, addition and/or conservative substitution and/or modified by known chemical methods, e.g. acetylation and/or esterification and/or functionalization of carboxy, hydroxy, thio or amino groups present on the amino acid side chain. Said derivatives or analogues are obtained by well known methods aiming at improving the metabolic stability of the peptides.

The peptides may be prepared by standard methods such as the solid-phase-9-fluorenylmethoxycarbonyl (Fmoc) method using conventional apparatuses, reagents, resins and protected amino acids.

In the following experimental sections, given by way of exemplification of the invention, the peptides having SEQ ID 3-10 are respectively identified as 1C, 1 N, 3C, 3N, 1I, 3I, 1CI, 3CI.

### Descriptions of the Figures

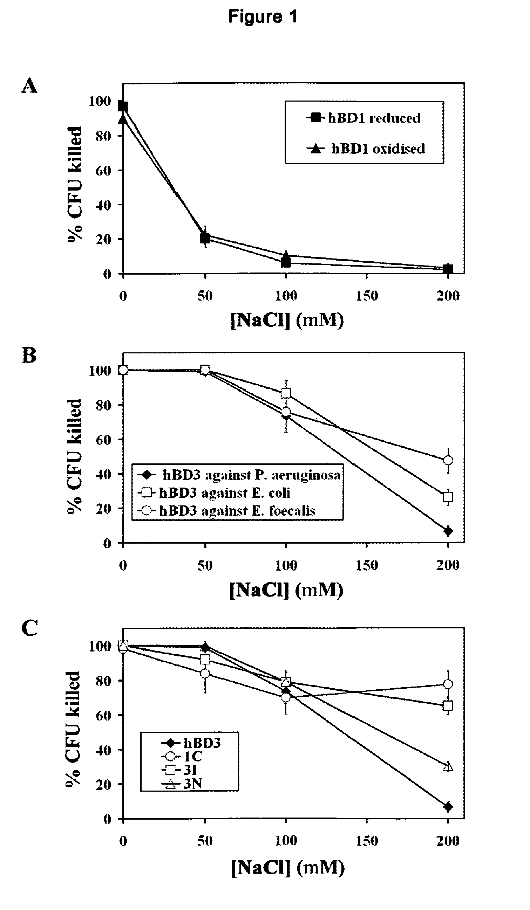

**Fig. 1****.** Antibacterial activity at increasing NaCl concentration of (panel A) reduced and oxidized wild-type hBD1 (2.5 µM) against *P. aeruginosa*; (panel B) wild type hBD3 (2.5 µM), against *P. aeruginosa, E. faecalis* and *E. coli*; (panel C) wild-type hBD3 and analogs 3N, 1C and 3I (2.5 µM) against *P. aeruginosa*. The SD of each replicate measurement is indicated by vertical bars through the symbols.

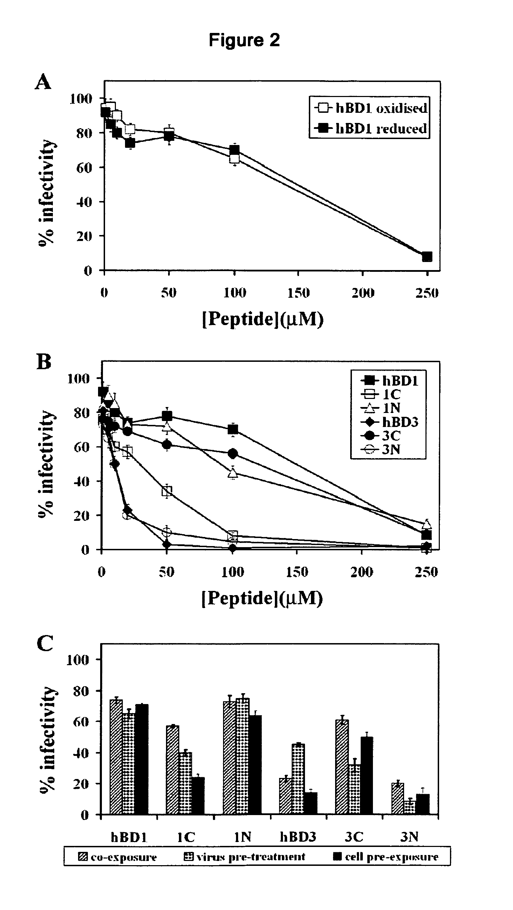

**Fig. 2****.** Antiviral activity of reduced and oxidized wild type hBD1 at different peptide concentrations (panel A); antiviral activity (virus and cell co-exposure) at increasing concentration of hBD1, hBD3, 1C, 1N, 3C and 3N analogs (panel B); antiviral activity of each peptide hBD1, hBD3, 1C, 1N, 3C and 3N analogs (20 µM) after different treatments (see Materials and Methods): co-exposure (virus, cell and peptide), virus plus peptide pre-treatment and cell plus peptide pre-exposure (panel C). The SD of each replicate measurement is indicated by vertical bars through the symbols.

### Example 1 - Peptide preparation

Peptides were synthesized using the standard solid-phase-9-fluorenylmethoxycarbonyl (Fmoc) method, on a PSSM8 multispecific peptide synthesizer (Shimadzu Corporation Biotechnology, Kyoto, Japan). The NovaSyn TGA (Merck, Darmstadt, Germany) resin (substitution 0.25 mmol/g) was used as solid-phase support, and syntheses were performed on a scale of 100 µmol. Ten equivalents of the first amino acid were coupled on the resin according to the N,N'-Dicyclohexylcarbodiimide (DIC)/4-Dimethylaminopyridine (DMAP) method (10 equivalents of Fmoc-amino acid, 5 equivalents of DIC, 0.1 equivalents of DMAP). All successive amino acids, 4 equivalents relative to resin loading, were coupled according to the N-hydroxybenzotriazole (HOBT)/2-Benzotriazole-3-tetramethyluronium tetrafluoroborate (TBTU)/Di-iso-propylethylamine (DIPEA) method [1 equivalent of Fmoc-amino acid, 1 equivalent of TBTU, 1 equivalent of HOBT (0.5 mM HOBT in NMP), 2 equivalents of DIPEA (1 mM DIPEA in N-methylpyrrolidone, NMP)]. The Fmoc protecting group was removed with 30% piperidine in NMP (v/v). Peptides were fully deprotected and cleaved from the resin with trifluoroacetic acid (TFA) with 5% thioanisole, 3% ethanedithiol and 2% anisole as scavengers. The crude peptides were precipitated with ice-cold ethyl ether, filtered, dissolved in water, lyophilized, and reduced with dithiothreitol (DTT). Peptides were purified to homogeneity by preparative reverse phase-high pressure liquid chromatography (RP-HPLC) on a Schimadzu Class-LC8A chromatographic system, equipped with an UV Lambda SPD-10A detector. The samples were injected on a Phenomenex (Phenomenex, Torrance, CA, USA) C₁₈ column (22 mm x 25 cm, 5 mm) eluted by H₂O/0.1% TFA (A) and CH₃CN/0.1% TFA (B) solvent mixture. A linear gradient from 5 to 50% of B over 17 min at a flow rate of 20 mL/min was used. The collected fractions were lyophilized to dryness and analyzed by analytical reverse-phase HPLC on a Shimadzu class-LC10 equipped with a diode array detector SPD-M10AV using a Phenomenex C₁₈ analytical column (4.6 x 250 mm, 5mm). The identity of purified peptides was confirmed by electron spray ionisation liquid chromatography-mass spectrometry liquid chromatography/mass spectrometry (ESI LC-MS) using a Thermo Electron MSQ Surveyor.

*Folding and disulphide formation*-Several protocols were used to determine the best condition for folding. 1) Air oxidation was performed by dissolving samples at 1mM in 50 mM phosphate buffer pH 6.0 or 7.7, followed by gentle stirring in an open air container; 2) oxidation in dimethyl sulfoxide (DMSO) 10%, 15% or 20% was performed by dissolving samples in three different buffers at 1mM peptide concentration (i. 50 mM phosphate buffer pH 6, 1mM ethylenediaminetetracetic acid (EDTA); ii. 5% acetic acid pH 6.0; iii. 0.1 M TrisHCl pH 8.2, 1 mM EDTA); 3) oxidation with reduced and oxidized glutathione (5/1 or 10/1) was performed by dissolving samples at 1 mM in a solution containing reduced and oxidized glutathione, in two different buffers (i. 50 mM phosphate buffer pH 6, 1mM EDTA, ii. 0.1M TrisHCl pH 7.7, 1 mM EDTA). Folding reactions were monitored by RP (reverse phase)-HPLC at 8, 16 and 48 hours. All folded products were analyzed and purified, and their molecular masses were determined by LC-MS.

### Example 2 - Antimicrobial activity assay

Colony-forming unit (CFU) assay of the antibacterial activity of the peptides against *P.aeruginosa* ATCC (American Type Culture Collection, Manassas, VA, USA) 27853, *Enterococcus faecalis* ATCC 29212 and *Escherichia coli* ATCC 25922 was performed. The strains were grown in aerobic conditions in tryptic soy broth (Difco Laboratories, Detroit, MI, USA) at 37°C. Cells from exponential-phase cultures were centrifuged, washed and suspended to a density of 1 x 10⁵ CFU/mL in sterile 10 mM potassium phosphate buffer, pH 7.4, and incubated in the presence of the peptides. The incubation time was 2 h at 37°C. Two concentrations of peptides were used, i.e., 2.5 µM, at which 100% of *P. aeruginosa* strains are killed by the wild-type hBD3 (minimal bactericidal concentration; MBC) and 12.5 µM. For salt dependence assay, NaCl in a range of concentrations potentially present in the airway surface fluid of normal subjects and CF patients (33), i.e., 0, 50, 100 and 200 mM NaCl, were included in the incubation buffer. The cells were then diluted serially in the same buffer, spread on tryptone-yeast (Difco Laboratories) plates and the colonies were counted after 18 h at 37°C. Each assay was performed in triplicate. Bactericidal activity (mean and SD of three assays) is expressed as the ratio between colonies counted and the number of colonies on a control plate.

The results of the antibacterial activity of the wild-type hBDs and analogs against *P. aeruginosa, E. coli* and *E. faecalis* expressed as per cent of killed CFU are reported in Tables 1-3, respectively. As reported in Table I, at a concentration of 2.5 µM of the peptide, the antibacterial activity of hBD3 against *P. aeruginosa* was unaffected by 50 mM NaCl, slightly inhibited by 100 mM NaCl (paired t test versus hBD3 activity at 0 mM NaCl: p<0.01) and greatly inhibited by 200 mM NaCl (paired t test versus hBD3 activity at 0 mM NaCl: p<0.001). At a concentration of 12.5 µM of peptide, the antibacterial activity of hBD3 was strongly inhibited only by 200 mM NaCl (unpaired t test versus hBD3 activity at 0 mM NaCl: p<0.001). Similarly, at 200 mM NaCl, the antibacterial activity of hBD3 against *E. coli* (Table II) and *E. faecalis* (Table III) was significantly inhibited compared to the activity in the absence of salt, but it was significantly higher (paired t test: p<0.01 in both cases) than that displayed by hBD3 against *P. aeruginosa* at 200 mM NaCl. This is also true at peptide concentrations of 2.5 µM (see also Fig. 1 B) and 12.5 µM.

The antibacterial activity of wild-type hBD1 and analogs was compared to the results obtained with wild-type hBD3 at the same concentrations of peptide and levels of NaCl. The antibacterial activity of wild-type hBD1, and analogs 1N, 1I and 3CI against *P. aeruginosa* (Table I), *E. coli* (Table II) and *E. faecalis* (Table III) was inhibited already by 50 mM NaCl. However, at a concentration of 12.5 µM of the peptide, the antibacterial activity of analogs 1N and 3CI remained significantly higher (p<0.01) compared to wild-type hBD3 in the presence of 200 mM NaCl. The antibacterial activity of analog 1C versus *P. aeruginosa* was similar to that of wild-type hBD3 at 0, or 50 or 100 mM of NaCl, whereas it was significantly higher (unpaired t test versus wild-type hBD3 under the same conditions: p<0.001) at 200 mM NaCl (Table I); this is true at concentration of both 2.5 and 12.5 µM of the peptide. The same pattern of activity was observed against *E. coli* (Table II), and *E. faecalis* (Table III). Finally, analogs 3N, 1CI and 31 displayed antibacterial activity at 200 mM NaCl against all three bacteria, as measured by unpaired t test versus hBD3 in the same conditions: p<0.001 (see Tables I-III and Fig. 1 C).

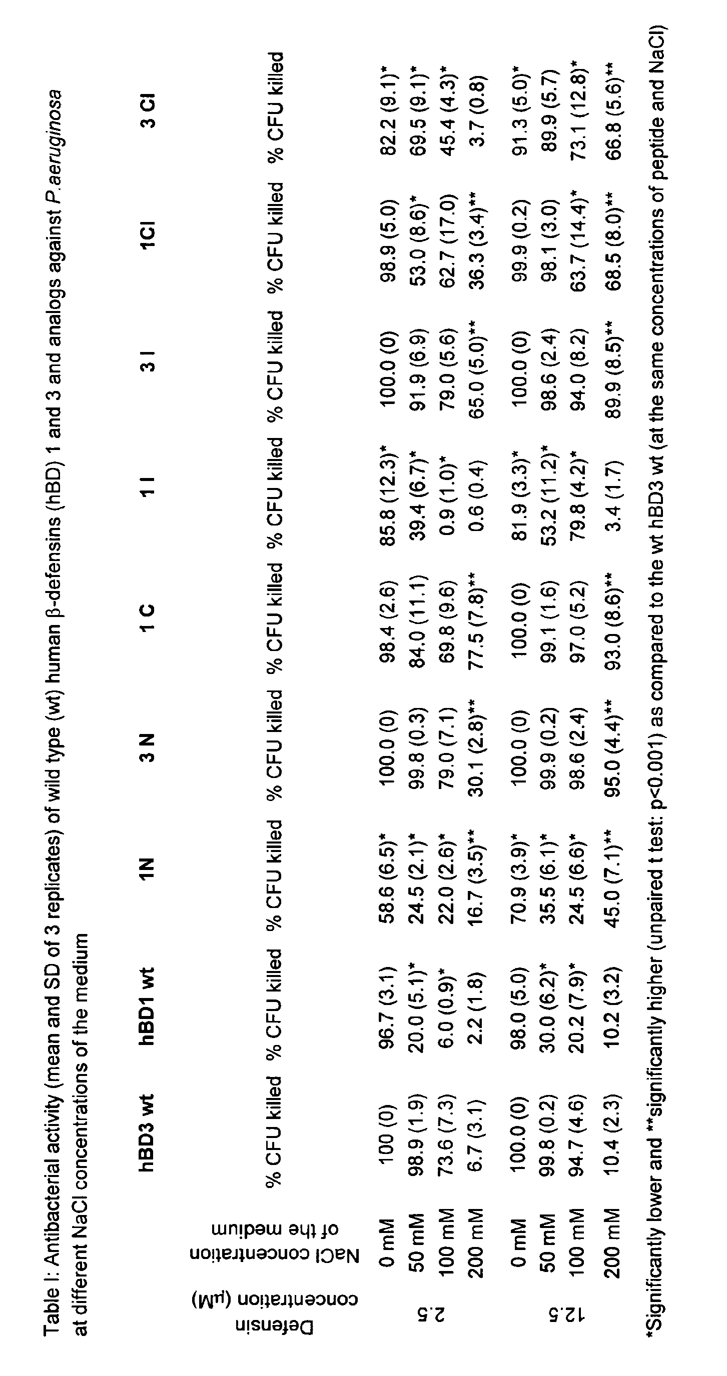

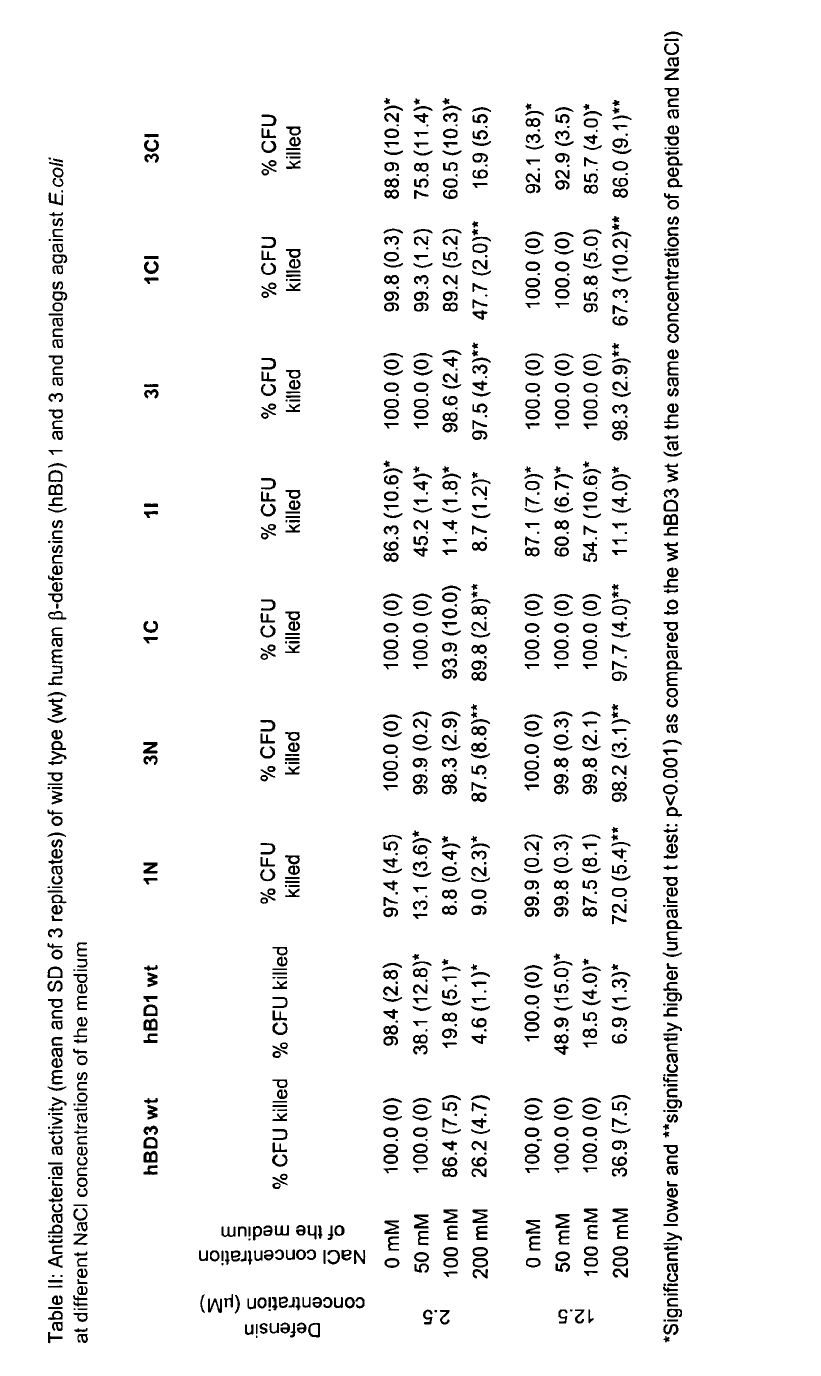

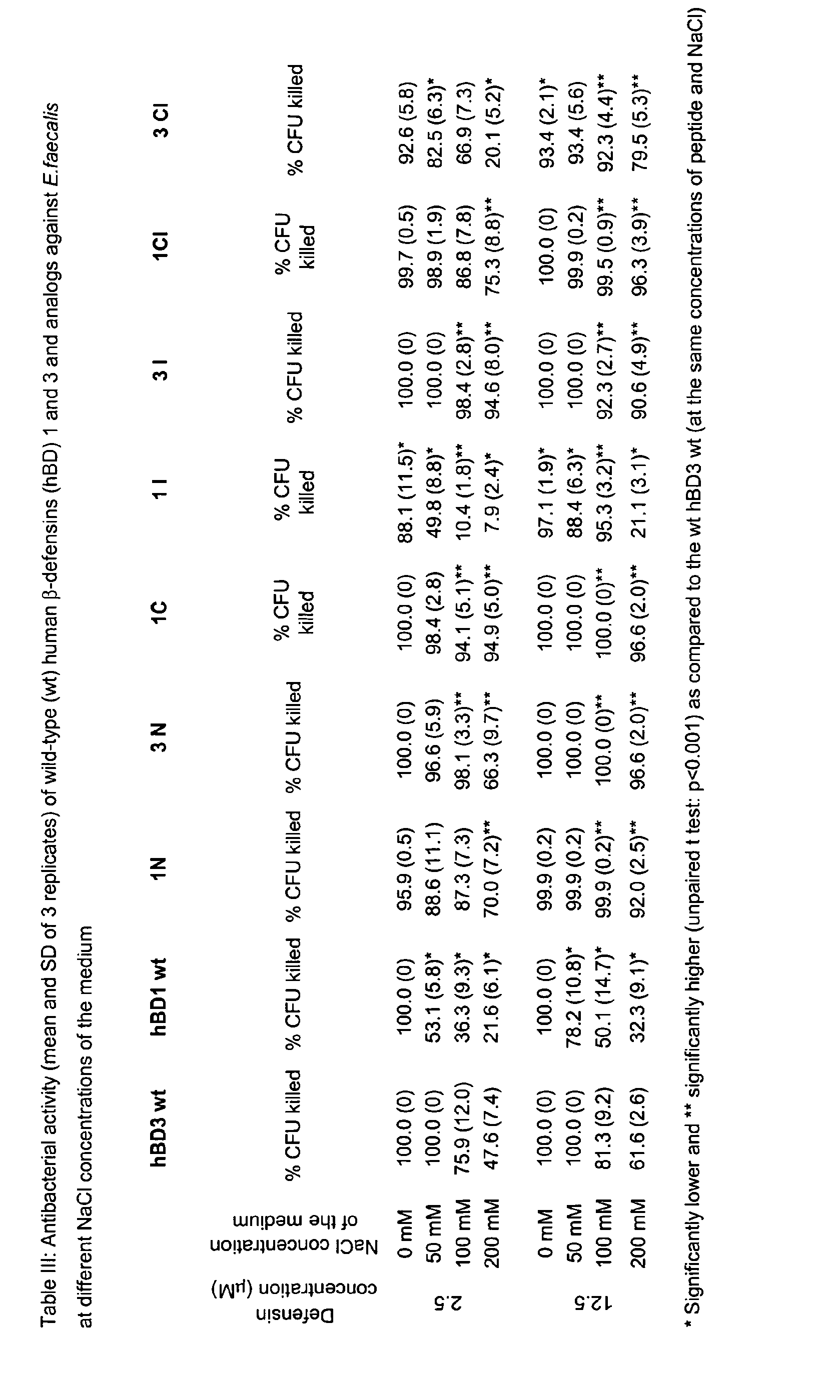

### Example 3 - Chemotactic activity

*Preparation of* leukocytes-Monocytes and neutrophils were isolated as described in the StemSep Cell Technical Manual (Stem Cell Technologies, Vancouver, Canada). Briefly, to obtain neutrophils, buffy coats were diluted 1:20 in NH4Cl supplemented with 2 mM EDTA and incubated on ice for 20 min. Cell suspensions were centrifuged for 10 min at 600 X g at room temperature with the brake off and washed three times with phosphate buffered saline (PBS) supplemented with 2% foetal bovine serum and 2 mM EDTA. Cell viability was assessed by analysis of light scatter properties, and cell count was performed using a logical gate strategy based on Trucount tubes (Becton-Dickinson, San Jose, CA, USA). Neutrophils were purified from lysed buffy coats using StemSep negative selection system. To this purpose, 100x106 mononuclear cells from lysed buffy coats were incubated with 100 µL of custom antibody cocktail for human granulocytes enrichment (Stem Cell Technologies) for 45 min on ice. In addition, cell suspensions were incubated with 60 µL of magnetic colloid for 30 minutes on ice. Finally, the samples were run through a 0.3-inch column matrix in a negative selection system in which the unwanted cells were immunomagnetically labelled and bound to the magnetic column. Neutrophils, after the separation, were collected in the column flowthrough without antibody bound to their surface. In order to obtain monocytes, human buffy coats were layered over a Ficoll-Paque density gradient and spun at 600 X g for 30 min with the brake off. The mononuclear cell-rich fractions were washed three times with PBS supplemented with 2% foetal bovine serum (FBS) and 2 mM EDTA. Cell viability was assessed by analysis of light scatter properties, and cell count was performed using a logical gate strategy based on Trucount tubes (Becton-Dickinson). A custom antibody cocktail for human monocyte enrichment (Stem Cells Technologies) was used for immunomagnetic negative selection of monocytes. Mononuclear cells were incubated as above described for neutrophils. Both neutrophils and monocytes were resuspended in 5.0 mL of RPMI-1640 (Sigma-Aldrich, St Louis, MO, USA) without phenol red containing 10% heat-treated FBS. Calcein AM (Molecular Probes, Eugene, OR, USA) was added to the cell suspensions (5.0 mL) at a concentration of 5 µg/mL and the cells were incubated for 30 min at 37°C. Cell suspensions were washed twice with PBS and then resuspended in RPMI-FBS to the desired concentration.

*Measurement of leukocytes migration*-The chemotaxis assay of monocytes and neutrophils (90-95% of purity) was performed in 96-well disposable chemotaxis chambers (Neuroprobe, Gaithersburg, MD, USA) using polyvinyl-pyrrolidone-free polycarbonate membranes with an 8-mm pore size for monocytes and with a 5-mm pore size for neutrophils. Chemotaxis was measured by fluorescence detection, using the calcein fluorescence signal. The fluorescence was read in a microplate fluorescence reader (Victor 3 1420 Multilabel Counter, Perkin Elmer) with excitation/emission wave-length settings of 485/530 nm. We calculated the migration index as the ratio between the sample mean fluorescence (after subtraction of the negative control mean fluorescence)/positive control mean fluorescence. Each assay was performed in quadruplicate.

The chemotactic activity of wild-type hBD1 and hBD3 against neutrophils and against monocytes at 100, 150 and 200 mM NaCl is reported in Table IV, 100 mM NaCl being the minimal concentration for the assay medium. NaCl concentrations up to 200 mM did not significantly affect the chemotactic activity of hBD1 against neutrophils whereas concentrations from 100 to 150 mM significantly reduced (unpaired t test: p<0.001) the chemotactic activity of hBD3 against neutrophils (Table IVA). The chemotactic activity of both hBD1 and hBD3 against monocytes (Table IVB) was significantly increased (unpaired t test: p<0.001) by NaCl concentrations between 100 and 150 mM, whereas at 200 mM NaCl, the activity was comparable to that obtained at 100 mM NaCl.

All analogs exerted chemotactic activity against neutrophils (Table VA), and activity was higher at a peptide concentration of 1.25 µM than at higher concentrations. Therefore, we compared the activity of each peptide at 1.25 µM to that exerted by 1.25 µM wild-type hBD1. Wild-type hBD3, and analogs 1N, 1I and 1CI had significantly lower activity than hBD1 (unpaired t test: p<0.001). The activity of 1C and 3N was comparable to that of hBD1 and finally the activity of the 3CI analog was significantly higher than that of wild-type hBD1. At peptide concentration higher than 1.25 mM (see Table VA) the chemotactic activity toward neutrophils and monocytes was dramatically reduced.

All peptides exerted a chemotactic effect on monocytes, with the greatest effect at peptide concentrations at 1.25 or 2.5 µM (Table VB). Also in this case, the chemotactic activity of each peptide at 1.25 µM was compared to that of wild-type hBD1 at the same concentration. The chemotactic activity of peptides 3CI, 1C and 1CI was significantly lower than that of hBD1 (unpaired t test: p<0.001). The activity of 1N and 3I was comparable to that of wild-type hBD1 and finally the activity of the wild-type hBD3 was significantly higher than that of wild-type hBD1 (unpaired t test: p<0.001).

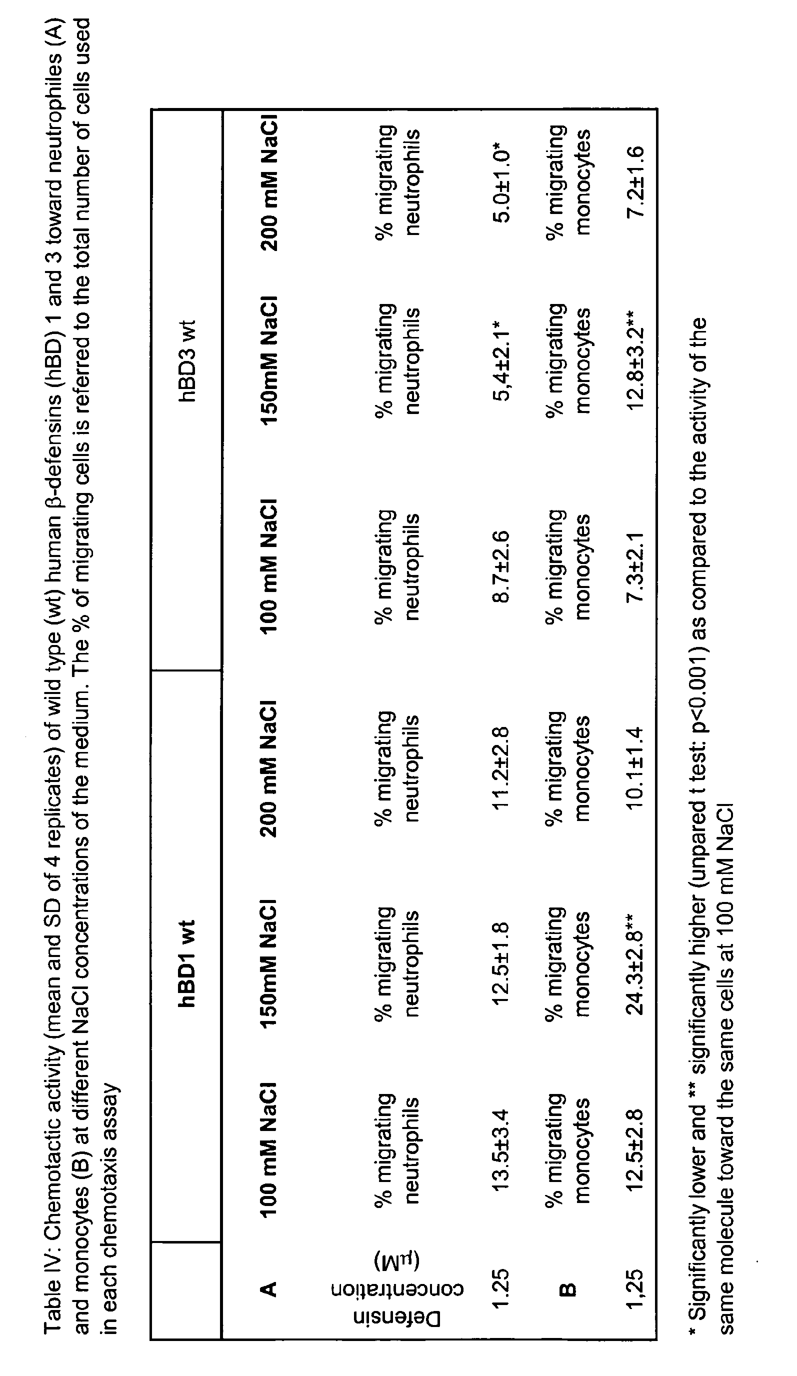

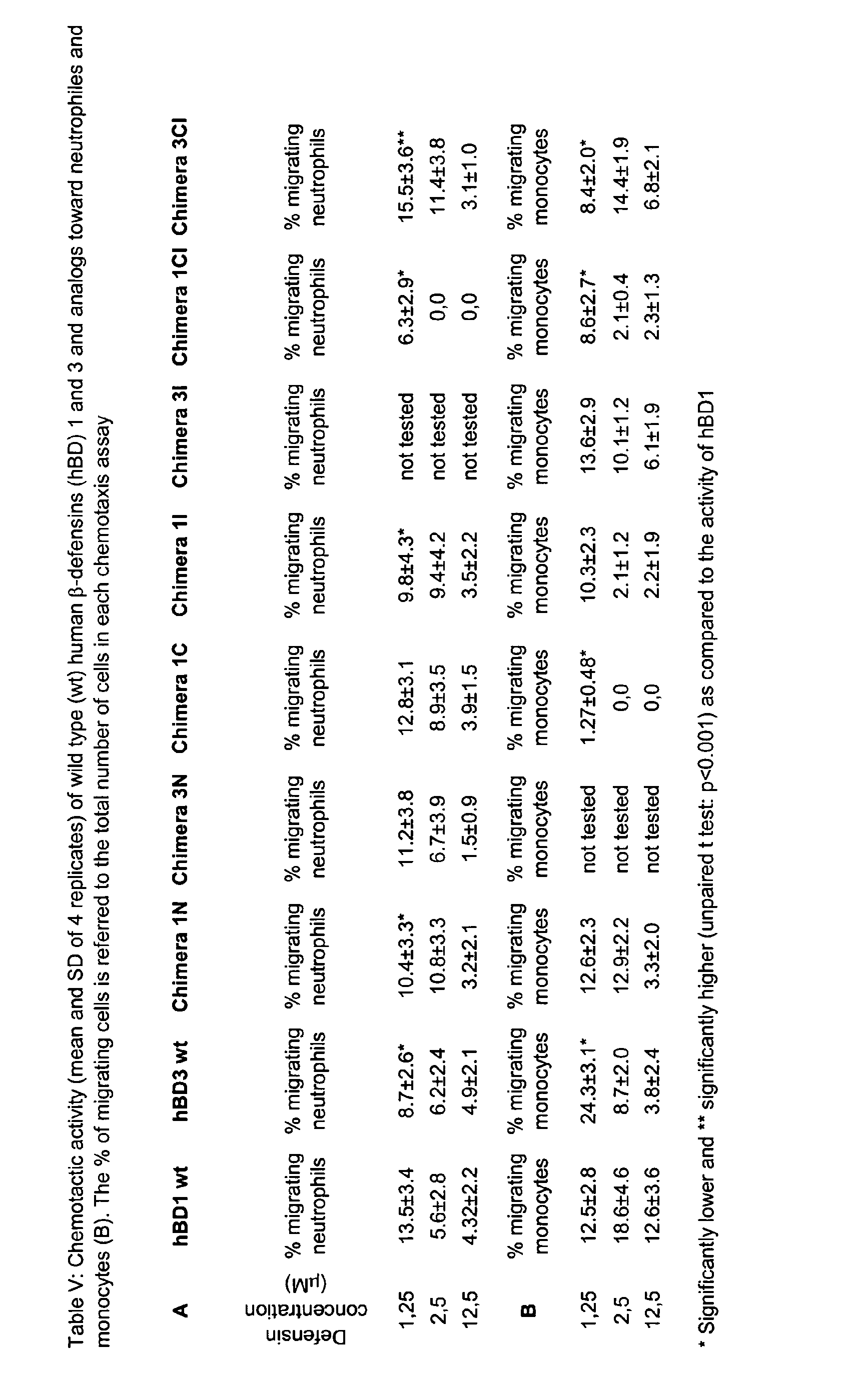

### Example 4 - Antiviral activity

Vero cells were grown in Dulbecco's modified Eagle's medium (DMEM) supplemented with 10% foetal calf serum. HSV-1 carrying a *IacZ* gene driven by the cytomegalovirus (CMV) IE-1 promoter to express beta-galactosidase was propagated as described (33). Peptides were dissolved in DMEM with serum and used at different concentrations. All experiments were conducted in parallel with scrambled peptides and no-peptide controls. To assess the effect of peptides on inhibition of HSV infectivity, cell monolayers were treated as follows. i) For "co-exposure" experiments, the cells were incubated with increasing concentrations of the peptides (1, 5, 10, 20, 50, 100, 250 µM) and with the viral inoculum for 45 min at 37°C. Non-penetrated viruses were inactivated by citrate buffer at pH 3.0 after the 45 min incubation with cells at 37°C. The cells were then incubated for 48 h at 37°C in DMEM supplemented with carboxymethylcellulose (CMC). Monolayers were fixed, stained with 5-bromo-4-chloro-3-indolyl-beta-D-galactopyranoside (X-gal) and plaque numbers were scored. Experiments were performed in triplicate and the percentage of inhibition was calculated with respect to no-peptide control experiments. ii) For "virus pre-treatment" experiments, approximately 2 x 10⁴ plaque forming units (PFU) of HSV-1 were incubated in the presence of 20 µM of peptides for 45 min at 37°C, then titrated on Vero cell monolayers. iii) For "cell pre-exposure" experiments, Vero cells were incubated with 20 µM of peptides for 30 min at 4°C. Peptides were removed, and cells were washed with phosphate-buffered saline before being infected with serial dilutions of HSV-1 and incubated for 45 min at 37°C.

Before testing the antiviral effect of hBDs and their analogs we verified that peptides did not exert toxic effects on Vero cells. Monolayers were exposed to different concentrations (10, 50, 100, 250 and 500 µM) of each peptide for 24 h, and cell viability was evaluated with the LDH assay. There was no significant difference between the viability of cells exposed to the peptides and that of cells exposed to control (data not shown). Thus, we evaluated the antiviral activity of wild type peptides and their analogs in: co-exposure experiments (cell + virus + peptide at zero time); virus pre-treatment (virus + peptide incubation before cell infection); and cell pre-exposure (cell + peptide incubation before virus infection). This would allow to evaluate if, and in which phase, the peptides could inhibit the virus infectivity.

For co-exposure experiments, we incubated the peptides on the monolayers of Vero cells together with the virus inoculum for 45 min before low pH treatment. The reduced form of wild-type hBD1 and hBD3 and analogs 1C, 1 N, 3C, 3N were used in all subsequent experiments. All peptides had a dose-dependent effect on HSV infectivity in the co-exposure experiment (Fig. 2B). The most active peptides were wild-type hBD3 (100% inhibition at 50 µM), analog 3N (90% inhibition at 50 µM) and analog 1C (70% inhibition at 50 µM). No control (peptide excluded) inhibited HSV-1 infectivity under similar experimental conditions.

We used peptide concentration of 20 µM to assess the effect of the three different treatments as described above (i.e., co-exposure, virus pre-treatment and cell pre-exposure). All peptides inhibited HSV infectivity when present during the period of virus attachment-entry into cells. The most efficient peptides were wild-type hBD3 and 3N (Fig. 2C and Table VI) whereas analog 1C was active when tested in cell pre-exposure treatment. Viral infectivity was not affected in any control experiment.

**Table VI: Antiviral activity (mean and SD of 3 replicates) of wild type (wt) human β-defensins (hBD) 1 and 3 and 1N, 1C, 3N and 3C analogs (20 µM) following different treatments: co-exposure, virus pre-incubation; cell pre-exposure**

| | **hBD3 wt** | **hBD1 wt** | **1N** | **3N** | **1C** | **3C** |
|---|---|---|---|---|---|---|
| | % of infectivity | % of infectivity | % of infectivity | % of infectivity | % of infectivity | % of infectivity |
| co-exposure | 23.4±2.5 | 74.7±2.4* | 73.2±4.2* | 20.4±2.4 | 57.6±1.8* | 61.7±3.4* |
| virus pre-incubation | 45±1.5 | 65.3±3.8* | 75.6±3.5* | 8.5±2.1** | 40.3±2.6 | 32.4±4.3 |
| cell pre-exposure | 14.5±2.3 | 71.3±1.2* | 64.8±3.5* | 13.4±4.2 | 24.1±2.2 | 50.3±3.4* |

| | | | | | | |
|---|---|---|---|---|---|---|
| * Significantly less and ** significantly more (unpaired t test <0.001) active as compared to wt hBD3 | | | | | | |

### REFERENCES

1) Pazgier, M., et al., Hoover, J.(2006) Cell. Mol. Life Sci. 63, 1294-1313
2) Schutte, et al., (2002) Proc. Natl. Acad. Sci. USA 99, 2129-2133
3) Rodriguez-Jimenez, F.J., et al., (2003) Genomics 81, 175-183
4) Vankeerberghen, A., et al., (2005) Genomics 85, 574-581
5) Hoover, D.M., et al., (2000) J. Biol. Chem. 275, 32911-32918
6) Bauer, F., et al., (2001) Protein Sci. 10, 2470-2479
7) Hoover, D.M., et al., (2001) J. Biol. Chem. 276, 39021-39026
8) Sawai, M.V., et al., (2001) Biochemistry 40, 3810-3816
9) Schibli, D.J., et al., (2002) J. Biol. Chem. 277, 8279-8289
10) Ganz, T. (2003) Nat. Rev. Immunol. 3, 710-720
11) Selsted, M.E., and Ouellette, A.J. (2005) Nat. Immunol. 6, 551-557
12) Lehrer, R.I. (2004) Nat. Rev. Microbiol. 2, 727-738
13) Goldman, M.J., et al., (1997) Cell 88, 553-560
14) Tomita, T., et al., (2000) Microbiol. Immunol. 44,749-754
15) Conejo-Garcia, et al., (2001) FASEB. J. 15, 1819-1821
16) Harder, J., et al., (2001) J. Biol. Chem. 276, 5707-5713
17) Starner, T.D., et al., (2005) J. Immunol. 174, 1608-1615
18) Smith, J.J., et al., (1996) Cell. 85, 229-236
19) Ratjen, F., and Doring, G. (2003) Lancet. 361, 681-689
20) Rosenstein, B.J., and Zeitlin, P.L. (1998) Lancet 351, 277-282
21) Oliver, A., et al., (2000) Science 288, 1251-1254
22) Lyczak, J.B., et al., (2002) Clin. Microbiol. Rev. 15, 194-222
23) Tager, A.M., et al., (1998) Am. J. Respir. Cell. Mol. Biol. 19, 643-652
24) Conejo-Garcia, J.R., et al., (2001) Cell. Tissue Res. 306, 257-264
25) Niyonsaba, F., et al., (2004) Immunology 111, 273-281
26) Yang, D., et al., Science 286, 525-528
27) Sun, L., et al., (2005) J. Virol. 79, 14318-14329
28) Quinones-Mateau, et al., (2003) AIDS 17, 39-48
29) De Clercq, E. (2001) J. Clin. Virol. 22, 73-89
30) Daher, K.A., et al., (1986) J. Virol. 60, 1068-1074
31) Sinha, S., et al., (2003) Antimicrob. Agents Chemother. 47, 494-500
32) Yasin, B., et al., (2004) J. Virol. 78, 5147-5156
33) Forrester, A., et al., (1992) J. Virol. 66, 341-348
34) Schneider, J.J., et al., (2005) J. Mol. Med. 83, 587-595
35) Sahly, H., et al., (2003) Antimicrob. Agents Chemother. 47, 1739-1741
36) Maisetta, G., et al (2005) Antimicrob. Agents Chemother. 9, 1245-1248
37) Maisetta, G., et al., (2006) Antimicrob. Agents Chemother. 50, 806-809
38) Sahl, H.G., et al., (2005) J. Leukoc. Biol. 77, 466-475
39) Yadava, P., et al., (2006) Int. J. Antimicrob. Agents 28, 132-137
40) Braida, L., et al., (2004) AIDS 18, 1598-1600
41) Cole, A.M., and Lehrer, R.I. (2003) Curr. Pharm. Des. 9, 1463-1473

## Claims

1. Peptides having sequences corresponding to fragments of human beta defensins 1 and/or 3 having antibacterial and/or antiviral activity in the presence of high sodium chloride concentration.

2. Peptides according to claim 1 having sequences corresponding to fragments of human beta defensin 1.

3. Peptides according to claim 1 having sequences corresponding to fragments of human beta defensin 3.

4. Peptides according to claim 1 having sequences corresponding to fragments of human beta defensin 1 and to fragments of human beta defensin 3.

5. Peptides according to any one of claims from 1 to 4 comprising from 5 to 40 amino acids, preferably from 8 to 42 amino acids, even more preferably from 12 to 39 amino acids.

6. Peptides according to any one of claims from 1 to 5, wherein the fragments include sequences of the C terminal of human beta defensin 3 or of human beta defensin 1, and/or sequences from the internal region of human beta defensin 1.

7. Peptides according to any one of claims from 1 to 6, having the following sequences:
- DHYNCVSSGGQCLYSACPIFTKIQGTCYRGKRKCCRRKK
- GIINTLDHYNCVSSGGQCLYSACPIFTKIQGTCYRGKAKCCK
- GIINTLQKYYCRVRGGRCAVLSCLPKEEQIGKCSTRGAKCCK
- QKYYCRVRGGRCAVLSCLPKEEQIGKCSTRGRKCCRRKK
- DHYNCVSSGGQCLYSACLPKEEQIGKCYRGKAKCCK
- GIINTLQKYYCRVRGGRCAVLSCPIFTKIQGTCSTRGRKCCRRKK
- DHYNCVSSGGQCLYSACLPKEEQIGKCYRGKRKCCRRKK
- GIINTLQKYYCRVRGGRCAVLSCPIFTKIQGTCSTRGAKCCK
- GIINTLQKYYCRVRGGRCAVLSCLPKQQQIGKCSTRGRKCCRRKK
- DHYNCVSSGG
- GIINTLQKYYCRVRGGRCAVLS
- QKYYCRVRGGRCAVLS
- IFTKIQGTAKCCK
- AKCCK
- IFTKIQGTRKCCRRKK
- RKCCRRKK
- IFTKIQGTKCCRRKK
- IFTKIQGT.

8. Peptides according to claim 7 having the following sequences:
- DHYNCVSSGGQCLYSACPIFTKIQGTCYRGKRKCCRRKK
- QKYYCRVRGGRCAVLSCLPKEEQIGKCSTRGRKCCRRKK
- GIINTLQKYYCRVRGGRCAVLSCPIFTKIQGTCSTRGRKCCRRKK
- DHYNCVSSGGQCLYSACLPKEEQIGKCYRGKRKCCRRKK.

9. Pharmaceutical compositions comprising a peptide of claims 1-8 as the active ingredient.

10. The use of a peptide of claims 1-8 for the preparation of medicaments having antibacterial, antiviral and chemotactic activity.
